# EUROPÄISCHE PATENTSCHRIFT

(11) **EP 3 261 564 B1**
(45) Veröffentlichungstag und Bekanntmachung des Hinweises auf die Patenterteilung: **30.11.2022**
(21) Anmeldenummer: 16702073.4
(22) Anmeldetag: 27.01.2016
(51) Int. Cl.: A61B 17/70, A61B 17/88, A61B 34/20, A61B 90/00, A61B 34/10

(54) **MEDIZINISCHES INSTRUMENTARIUM UND VERFAHREN**
SET OF MEDICAL INSTRUMENTS, AND METHOD
INSTRUMENT MÉDICAL ET PROCÉDÉ

(30) Priorität: 26.02.2015 DE 102015102776
(43) Veröffentlichungstag der Anmeldung: 03.01.2018
(73) Patentinhaber: Aesculap AG, 78532 Tuttlingen (DE)
(72) Erfinder: BEGER, Jens, 78532 Tuttlingen (DE); KOZAK, Josef, 78532 Tuttlingen (DE); STOERK, Claudia, 78576 Emmingen (DE)
(74) Vertreter: Hoeger, Stellrecht & Partner Patentanwälte mbB
(86) Internationale Anmeldenummer: PCT/EP2016/051671
(87) Internationale Veröffentlichungsnummer: WO 2016/134911

(56) Entgegenhaltungen:
- EP-A1- 1 281 365
- EP-A1- 1 657 678
- EP-A1- 2 910 206
- WO-A1-01/59708
- WO-A2-2013/164770
- US-B2- 8 549 888

## Beschreibung

Die Erfindung betrifft ein medizinisches Instrumentarium.

Außerdem betrifft die Erfindung ein Verfahren zum Ermitteln der Form eines chirurgischen Stabilisierungselementes.

Chirurgische Stabilisierungselemente kommen insbesondere als Bestandteile von chirurgischen Fixationssystemen zum Einsatz. Mittels eines derartigen Fixationssystemes lassen sich beispielsweise Knochen oder Knochenstücke relativ zueinander fixieren; ein typisches Anwendungsgebiet ist die Wirbelsäulenchirurgie, bei der Wirbelkörper gegen Bewegung relativ zueinander gesichert werden sollen. Dabei werden in den Wirbelkörpern Verankerungselemente, beispielsweise Knochenschrauben, verankert und mittels des Stabilisierungselementes, beispielsweise eines Stabes, miteinander verbunden.

Ein derartiges Fixationssystem ist beispielsweise in der DE 10 2010 016 448 A1 beschrieben.

Um einen Eingriff möglichst geringer Invasivität vorzunehmen ist es wünschenswert festzustellen, ob das Stabilisierungselement dazu geeignet ist, die Verankerungselemente so miteinander zu verbinden, dass die gewünschte Fixation erzielt wird. Erforderlichenfalls kann die Form des Stabilisierungselementes geändert oder ein Stabilisierungselement aus einer Mehrzahl von zur Verfügung stehenden Stabilisierungselementen unterschiedlicher Form ausgewählt werden. Vorzugsweise erfolgt die Feststellung, die Formänderung und/oder die Auswahl vor der Implantation des Stabilisierungselementes.

In der DE 103 14 882 A1, der US 2005/0262911 A1 und der US 8,549,888 B2 sind jeweils Vorrichtungen beschrieben, mit denen die Form chirurgischer Stabilisierungselemente verändert werden kann. Bei der Vorrichtung der letztgenannten Druckschrift kommt ein computerimplementiertes Verfahren zum Einsatz, bei dem über eine graphische Benutzeroberfläche eine Biegekurve für das Stabilisierungselement vorgegeben werden kann, die an die Lage von Verbindungselementen für Knochenstrukturen angepasst ist.

Die WO 2013/164770 A2 beschreibt eine Ausführungsform eines handhaltbaren Trackingsystems zur Ausrichtung von Implantatssystemen während einer Operation.

Eine medizintechnische Befestigungsvorrichtung für eine Markiereinrichtung ist in der EP 1 281 365 A1 beschrieben.

Aufgabe der vorliegenden Erfindung ist es, ein Instrumentarium und ein Verfahren bereitzustellen, mit dem die Form eines chirurgischen Stabilisierungselementes auf einfache Weise ermittelt werden kann.

Diese Aufgabe wird erfindungsgemäß gelöst durch ein medizinisches Instrumentarium mit einem Navigationssystem, das eine eine Kamera aufweisende optische Erfassungseinheit aufweist und eine mit dieser gekoppelte Datenverarbeitungseinheit, mit einem Stabilisierungselement eines chirurgischen Fixationssystems sowie einer medizinischen Markiereinrichtung, die in definierter räumlicher Anordnung am Stabilisierungselement gehalten oder von diesem umfasst oder gebildet ist, wobei mit dem Navigationssystem die Lage und Orientierung der Markiereinrichtung im Raum ermittelbar ist, wobei mittels der Erfassungseinheit zumindest zwei Aufnahmen des Stabilisierungselementes und der Markiereinrichtung unter unterschiedlicher Orientierung erstellbar und die Form des Stabilisierungselementes von der Datenverarbeitungseinheit anhand der zwei oder mehr Aufnahmen ermittelbar ist.

In die vorliegende Erfindung fließt der Gedanke mit ein, dass mittels eines medizinischen Navigationssystems eine medizinische Markiereinrichtung hinsichtlich Lage und Orientierung und dementsprechend Lageänderung und Orientierungsänderung erfasst werden kann. Das Navigationssystem weist eine optische Erfassungseinheit mit mindestens einer Kamera auf, mit der Aufnahmen des Stabilisierungselementes einschließlich der daran angeordneten Markiereinrichtung erstellt werden können. Zumindest zwei Aufnahmen werden zeitlich aufeinanderfolgend unter unterschiedlicher Orientierung erstellt, d. h. unter unterschiedlicher relativer Lage und Richtung des Navigationssystems und des Stabilisierungselementes mit der Markiereinrichtung. Die Markiereinrichtung stellt eine Referenz bereit und definiert ein Referenzkoordinatensystem, in dem aus den Aufnahmen des Stabilisierungselementes von der Datenverarbeitungseinheit dessen Form erstellt werden kann. Hierzu können in der Datenverarbeitungseinheit Bildverarbeitungsalgorithmen ausführbar hinterlegt sein, die die Aufnahmen der mindestens einen Kamera analysieren und anhand der Lage- und/oder Orientierungsinformation aufgrund der Markiereinrichtung die dreidimensionale Gestalt des Stabilisierungselementes in hinreichender Genauigkeit bestimmen.

Das erfindungsgemäße Instrumentarium erlaubt es insbesondere, die Form des Stabilisierungselementes zu ermitteln, ohne dass dieses zu diesem Zweck räumlich fixiert sein muss. Der apparative Aufwand, um die Form des Stabilisierungselementes zu ermitteln, kann dadurch gering gehalten werden. Dies erleichtert auch die Handhabung des Instrumentariums und verringert den Aufwand, die Form zu bestimmen.

Vorteilhafterweise umfasst die Erfassungseinheit genau eine Kamera, um die konstruktive Ausgestaltung des Navigationssystems zu vereinfachen. Das Vorsehen einer Stereokamera ist nicht erforderlich.

Bei dem Stabilisierungselement kann es sich um ein im Körper verbleibendes Implantat handeln oder um ein nur vorübergehend verwendetes Probeimplantat, das auch als Werkzeug des Instrumentariums angesehen werden kann.

Das Stabilisierungselement kann insbesondere, wie erwähnt, ein Stab sein.

Das Stabilisierungselement kann aus einem Kunststoffmaterial oder aus einem Metall gefertigt sein.

Günstig ist es, wenn aufeinanderfolgend mehr als zwei Aufnahmen des Stabilisierungselementes und der Markiereinrichtung erstellbar sind, anhand derer die Form des Stabilisierungselementes ermittelbar ist, wobei die Orientierungen und Aufnahmen paarweise voneinander unterschiedlich sind. Dadurch lässt sich die Form des Stabilisierungselementes noch genauer ermitteln.

Weist das Stabilisierungselement eine Längserstreckung auf, beispielsweise bei Ausgestaltung als Stab, werden die Aufnahmen bevorzugt unter Ausrichtung einer optischen Achse der mindestens einen Kamera im Winkel und insbesondere quer zur Längsrichtung des Stabilisierungselementes aufgenommen. Zwischen zwei Aufnahmen wird die Kamera bevorzugt um 90° bezüglich der Längserstreckung des Stabilisierungselementes gedreht.

Für eine konstruktiv einfache Ausgestaltung und eine kostengünstige Fertigung des Instrumentariums ist es günstig, wenn das Navigationssystem ein handhaltbares integriertes Navigationssystem ist. Unter "integriert" wird vorliegend insbesondere verstanden, dass die Erfassungseinheit und die Datenverarbeitungseinheit in einem gemeinsamen Gehäuse angeordnet sind. In dem Gehäuse ist vorzugsweise eine Anzeigeeinheit des Navigationssystems angeordnet.

Beispielsweise ist das handhaltbare integrierte Navigationssystem ein Smartphone oder ein Tabletcomputer. Auf dem Smartphone oder Tabletcomputer kann ein Datenverarbeitungsprogramm ausführbar gespeichert sein, mit dem Daten der Erfassungseinheit von der Datenverarbeitungseinheit analysiert und die Form des Stabilisierungselementes ermittelt werden können.

Von Vorteil ist es, wenn das Navigationssystem eine mit der Datenverarbeitungseinheit gekoppelte Anzeigeeinheit umfasst, an der die Aufnahmen des Stabilisierungselementes darstellbar oder bereitstellbar sind und/oder Hinweise für einen Benutzer, die Aufnahmen durchzuführen und/oder eine Darstellung des Stabilisierungselementes, ermittelt anhand der Aufnahmen.

Es kann vorgesehen sein, dass das Stabilisierungselement Markierelemente der Markiereinrichtung umfasst oder bildet, beispielsweise sind die Markierelemente ausgestaltet als bevorzugt reflektierende Punkte oder Linien am Stabilisierungselement.

Das Navigationssystem kann eine Beleuchtungseinheit aufweisen, mit der Licht, insbesondere sichtbares Licht, in Richtung auf die Markiereinrichtung emittierbar ist. Die Beleuchtungseinheit umfasst zum Beispiel mindestens eine LED-Lichtquelle und wird vorzugsweise von dem integrierten, handhaltbaren Navigationssystem umfasst.

Die Markiereinrichtung kann am Stabilisierungselement mittelbar oder unmittelbar lösbar festgelegt oder festlegbar sein.

Bei einer vorteilhaften Ausführungsform des Instrumentariums ist die Markiereinrichtung einstückig mit dem Stabilisierungselement verbunden, beispielsweise an dieses angeformt oder angeschweißt.

Günstigerweise ist eine Sollbruchstelle vorgesehen zum Trennen der Markiereinrichtung vom Stabilisierungselement. Nach Ermitteln der Form des Stabilisierungselementes kann die Markiereinrichtung von diesem getrennt und das Stabilisierungselement implantiert werden.

Günstig ist es, wenn das Instrumentarium ein Implantationswerkzeug für das Stabilisierungselement aufweist, an dem dieses und die Markiereinrichtung gehalten sind. Das Implantationswerkzeug ist beispielsweise handgeführt und erlaubt es, das Stabilisierungselement bevorzugt perkutan und minimalinvasiv zu implantieren. Über das Implantationswerkzeug ist die Markiereinrichtung in definierter räumlicher Anordnung zum Stabilisierungselement angeordnet. Die Markiereinrichtung kann mit dem Implantationswerkzeug lösbar verbindbar sein. Vorliegende Ausführungsform erlaubt es, die Form des Stabilisierungselementes zu ermitteln. Zugleich ist die Möglichkeit gegeben, das Stabilisierungselement über das Implantationswerkzeug mit der daran gehaltenen Markiereinrichtung bei der Implantation zu verfolgen. Besonders bei perkutaner Implantation wird dadurch die Handhabung eines chirurgischen Fixationssystems erheblich vereinfacht.

Vorzugsweise weist das Instrumentarium eine Verformungseinrichtung auf, mit der die Form des Stabilisierungselementes veränderbar ist.

Die Verformungseinrichtung ist zum Beispiel eine Biegeeinrichtung zum Biegen eines Stabes, als der das Stabilisierungselement ausgestaltet ist.

Günstig ist es, wenn einer Bedienperson von der Datenverarbeitungseinheit an einer Hinweiseinheit des Navigationssystems, beispielsweise an einer Anzeigeeinheit, Formänderungsinformationen zur Handhabung der Verformungseinrichtung bereitstellbar sind, um das Stabilisierungselement ausgehend von der ermittelten Form in eine vorgebbare Form zu bringen. Beispielsweise kann die Datenverarbeitungseinheit vergleichen, ob die ermittelte Form des Stabilisierungselementes mit einer erforderlichen, gewünschten Form übereinstimmt. Die erforderliche Form kann zum Beispiel ermittelt werden, indem die Relativpositionen von Verankerungselementen des Fixationssystems festgestellt werden mit der Maßgabe, dass die Verankerungselemente mit dem Stabilisierungselement zu verbinden sind. Weicht die anhand der Aufnahmen ermittelte Form von der erforderlichen Form ab, kann das Stabilisierungselement mit der Verformungseinrichtung verformt werden. Zu diesem Zweck können der Bedienperson an der Anzeigeeinheit Formänderungsinformationen bereitgestellt werden und dadurch die Handhabung des Instrumentariums und des Fixationssystems erheblich vereinfacht werden.

In entsprechender Weise ist es günstig, wenn vom Navigationssystem über eine Kommunikationsschnittstelle Formänderungsinformationen an die Verformungseinrichtung übertragbar sind, um das Stabilisierungselement ausgehend von der ermittelten Form in eine vorgebbare Form zu bringen. Die Verformungseinrichtung kann das Stabilisierungselement vorzugsweise ohne Zutun der Bedienperson anhand der ihr zugeführten Formänderungsinformationen in die vorgebbare, erforderliche Form bringen.

Insbesondere ist denkbar, dass die Form des Stabilisierungselementes in situ erfasst und/oder bevorzugt in situ mittels der Verformungseinrichtung verändert wird.

Günstig ist es, wenn das Instrumentarium eine weitere medizinische Markiereinrichtung umfasst, die in definierter räumlicher Anordnung an der Verformungseinrichtung gehalten oder von dieser umfasst oder gebildet ist, wenn mit dem Navigationssystem die Lage und Orientierung der weiteren Markiereinrichtung ermittelbar ist und wenn einer Bedienperson mit der Datenverarbeitungseinheit an einer Hinweiseinheit des Navigationssystems, beispielsweise an einer Anzeigeeinheit, Hinweise zum Führen der Verformungseinrichtung bereitstellbar sind. Dies gibt die Möglichkeit, die Verformungseinrichtung mittels des Navigationssystems im Raum zu verfolgen. Dadurch kann der Verformungsvorgang überwacht werden, zum Beispiel eine Biegeebene oder ein Biegeradius.

Wie bereits erwähnt, betrifft die vorliegende Erfindung auch ein Verfahren. Die eingangs gestellte Aufgabe wird erfindungsgemäß durch ein Verfahren zum Ermitteln der Form eines chirurgischen Stabilisierungselementes unter Einsatz eines Instrumentariums der vorstehend genannten Art gelöst mit einem Navigationssystem, das eine eine Kamera aufweisende optische Erfassungseinheit aufweist und eine mit dieser gekoppelte Datenverarbeitungseinheit, mit einem Stabilisierungselement eines chirurgischen Fixationssystems sowie einer medizinischen Markiereinrichtung, die in definierter räumlicher Anordnung am Stabilisierungselement gehalten oder von diesem umfasst oder gebildet ist, wobei mit dem Navigationssystem die Lage und Orientierung der Markiereinrichtung im Raum ermittelbar ist, wobei mittels der Erfassungseinheit zumindest zwei Aufnahmen des Stabilisierungselementes und der Markiereinrichtung unter unterschiedlicher Orientierung erstellt werden und die Form des Stabilisierungselementes von der Datenverarbeitungseinheit anhand der zwei oder mehr Aufnahmen ermittelt wird.

Die nachfolgende Beschreibung einer bevorzugten Ausführungsform der Erfindung dient im Zusammenhang mit der Zeichnung der näheren Erläuterung der Erfindung. Es zeigen:
- Figur 1:: eine schematische perspektivische Darstellung eines erfindungs-gemäßen Instrumentariums zur Durchführung eines vorteilhaften Ausführungsbeispiels des erfindungsgemäßen Verfahrens und
- Figur 2:: eine Verformungseinrichtung des Instrumentariums aus Figur 1.

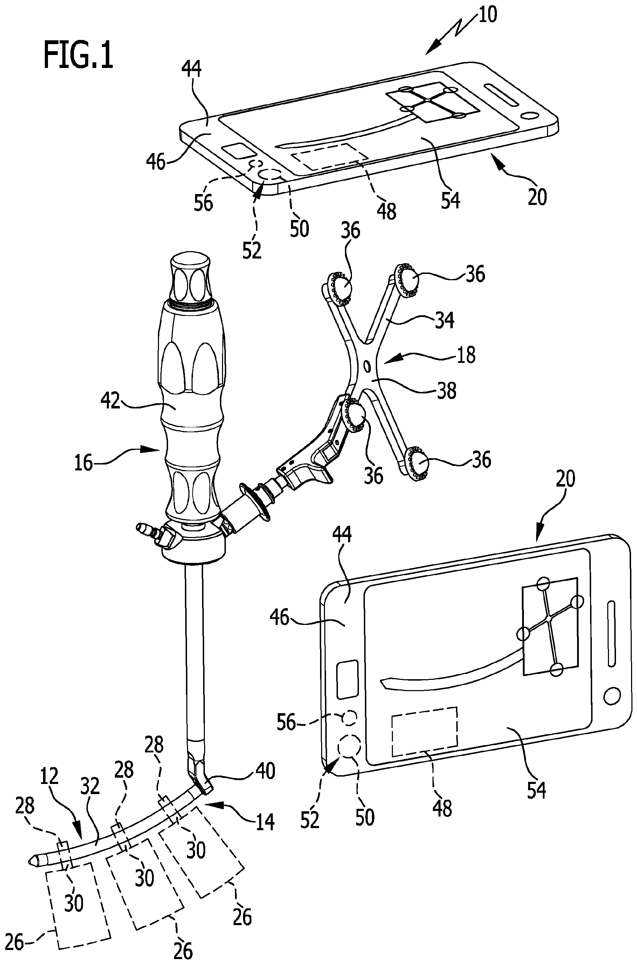

Figur 1 zeigt in perspektivischer Darstellung eine mit dem Bezugszeichen 10 belegte vorteilhafte Ausführungsform eines medizinischen Instrumentariums. Das Instrumentarium 10 umfasst ein Stabilisierungselement 12 eines chirurgischen Fixationssystems und ein Implantationswerkzeug 16 für das Stabilisierungselement 12. Weiter weist das Instrumentarium 10 eine medizinische Markiereinrichtung 18 auf und ein medizintechnisches Navigationssystem 20. Dieses ist in Figur 1 in zweierlei Orientierungen relativ zum Stabilisierungselement 12 dargestellt, beim Instrumentarium 10 jedoch nur einmal vorhanden.

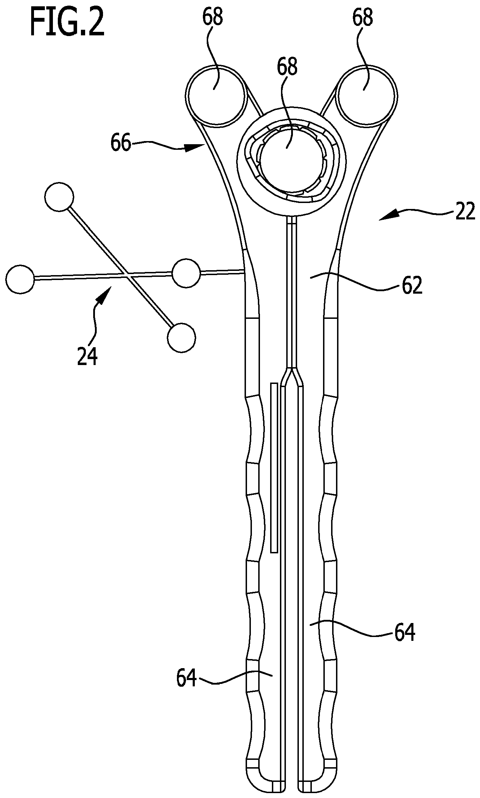

Das Instrumentarium 10 umfasst ferner eine in Figur 2 in Seitenansicht dargestellte Verformungseinrichtung 22 zum Verformen des Stabilisierungselementes 12 und eine daran angeordnete weitere medizinische Markiereinrichtung 24.

Das Fixationssystem 14 dient zur Fixierung von in Figur 1 schematisch dargestellten Wirbelkörpern 26 gegen Bewegung relativ zueinander. Zu diesem Zweck weist das Fixationssystem 14 Verankerungselemente 28 auf in Gestalt von Knochenschrauben 30, die an den Wirbelkörpern 26 fixiert sind. In Anwendung des Fixationssystems 14 wird das Stabilisierungselement 12, ausgestaltet als Stab 32, an den Knochenschrauben 30 fixiert. Über den Stab 32 sind die Knochenschrauben 30 starr miteinander verbunden.

Um die erforderliche Relativposition der Knochenschrauben 30 und damit der Wirbelkörper 26 sicherzustellen ist es in der Praxis von Bedeutung, dass der Stab 32 eine gewünschte, definierte Form aufweist. Die Form des Stabes 32 wird anhand des Navigationssystems 20 wie nachfolgend erläutert ermittelt.

Die medizinische Markiereinrichtung 18 ist vorliegend als sogenannter Rigid Body 34 ausgestaltet. Die Markiereinrichtung 18 umfasst eine Mehrzahl von Markierelementen 36. Die Markierelemente 36 sind an einer gemeinsamen Halterung 38 gehalten. Vorzugsweise sind die Markierelemente 36 retroreflektierend für insbesondere sichtbares Licht ausgebildet.

Die Markiereinrichtung 18 ist beim Instrumentarium 10 in definierter räumlicher Anordnung mittelbar am Stab 32 gehalten. Zu diesem Zweck dient das Implantationswerkzeug 16. Der Stab 32 ist beispielsweise an einem distalen Ende 40 des Implantationswerkzeuges 16 gehalten und nimmt relativ zu diesem eine definierte räumliche Anordnung ein. Die Markiereinrichtung 18 ist ebenfalls in definierter räumlicher Anordnung am Implantationswerkzeug 16 gehalten, beispielsweise an oder nahe einem Griffelement 42 des Implantationswerkzeugs 16.

Aus diesem Grund ist die räumliche Lage und Orientierung des Stabes 32 relativ zur Lage und Orientierung der Markiereinrichtung 18 bekannt. Wird die Markiereinrichtung 18 im Raum mittels des Navigationssystems 20 verfolgt, kann dadurch auch auf die Lage und die Orientierung des Stabes 32 geschlossen werden.

Zur erleichterten Handhabung und zur einfachen Ausgestaltung des Instrumentariums 10 ist das Navigationssystem 20 vorliegend als handhaltbares, integriertes Navigationssystem ausgestaltet. Beispielsweise handelt es sich dabei um ein(en) Smartphone oder Tablet-Computer 44. "Integriert" ist vorliegend insbesondere dahingehend aufzufassen, dass die Komponenten des Navigationssystems 20 in einem gemeinsamen Gehäuse 46 des Navigationssystems 20 angeordnet sind. Beispielsweise weist das Navigationssystem 20 eine im Gehäuse 46 angeordnete Datenverarbeitungseinheit 48 auf.

Ferner ist eine eine Kamera 50 aufweisende optische Erfassungseinheit 52 im Gehäuse 46 angeordnet. Günstigerweise ist genau eine digitale Kamera 50 vorgesehen. Im Gehäuse 46 ist außerdem eine Hinweiseinheit angeordnet, ausgestaltet als Anzeigeeinheit 54. Die Anzeigeeinheit 54 ist insbesondere ein Touchscreen.

Ferner umfasst das Navigationssystem 20 eine im Gehäuse 46 angeordnete Beleuchtungseinheit 56, die insbesondere eine LED-Lichtquelle umfasst. Mit der Beleuchtungseinheit 56 kann Licht, insbesondere sichtbares Licht, in Richtung der Markiereinrichtung 18 emittiert werden, und von deren Markierelementen 36 reflektiertes Licht kann von der Kamera 50 empfangen werden.

Die Datenverarbeitungseinheit 48 umfasst zum Beispiel einen Mikroprozessor oder ist als solcher ausgestaltet, auf dem ein Anwendungsprogramm des Navigationssystems 20 ausgeführt werden kann. Das Anwendungsprogramm umfasst insbesondere Algorithmen für die Bildverarbeitung.

Beim Instrumentarium 10 kann mit dem Navigationssystem 20 die Form des Stabes 32 auf einfache Weise ermittelt werden. Zu diesem Zweck kann die Bedienperson mit der Kamera 50 eine erste Aufnahme der Markiereinrichtung 18 und des Stabes 32 erstellen. Dies entspricht beispielsweise der Anordnung des Navigationssystems 20 unten in Figur 1.

Anschließend kann die Bedienperson eine zweite Aufnahme der Markiereinrichtung 18 und des Stabes 32 in geänderter Orientierung erstellen. Dies entspricht zum Beispiel der oberen Darstellung des Navigationssystems 20 in Figur 1.

Es ist denkbar, dass die Bedienperson von der Markiereinrichtung 18 und dem Stab 32 weitere Aufnahmen erstellt, wobei die Aufnahmen und die jeweilige Orientierung paarweise voneinander unterschiedlich sind.

Die Datenverarbeitungseinheit 48 ist so programmiert, dass sie anhand der von der Kamera 50 erstellten, zeitlich aufeinanderfolgenden Aufnahmen die Form des Stabes 32 ermittelt. Dies ist dadurch möglich, dass aufgrund der bekannten räumlichen Anordnung der Markiereinrichtung 18 zum Stab 32 durch Lage und Orientierung der Markiereinrichtung 18 relativ zur Kamera 50, wie vorstehend erläutert, auch auf Lage und Orientierung des Stabes 32 geschlossen werden kann.

Es zeigt sich in der Praxis, dass bereits mit zwei Aufnahmen durch Bildverarbeitung eine hinreichend gute Bestimmung der Form des Stabes 32 in drei Dimensionen möglich ist.

Vorzugsweise können der Bedienperson an der Anzeigeeinheit 54 Hinweise bereitgestellt werden, dass und wie, insbesondere in welcher Orientierung, die Aufnahmen erstellt werden sollten. Dabei ist es günstig, dass bei einem eine Längserstreckung aufweisenden Stabilisierungselement 12 eine optische Achse der Kamera 50 ungefähr quer zur Längserstreckung des Stabilisierungselementes 12 ausgerichtet ist.

In Figur 1 wird dies dadurch schematisch dargestellt, dass das Navigationssystem 20 zum Erstellen der zwei Aufnahmen um etwa 90° in Bezug auf den Stab 32 rotiert wird.

An der Anzeigeeinheit 54 können auch die Aufnahmen selbst oder eine Darstellung des Stabilisierungselementes 12, ermittelt anhand der Aufnahmen, dargestellt werden. Dies ist in Figur 1 schematisch gezeigt.

Ist die Form des Stabes 32 ermittelt, kann die Datenverarbeitungseinheit 48 feststellen, ob der Stab 32 die erforderliche Form und insbesondere Krümmung aufweist, damit die Knochenschrauben 30 wie vorgesehen miteinander verbunden werden können. Ist dies der Fall, kann der Stab 32 mit dem Implantationswerkzeug 16 implantiert werden.

Als besonders vorteilhaft erweist es sich dabei, dass die Markiereinrichtung 18 am Implantationswerkzeug 16 festgelegt ist. Unter Verfolgung der Markiereinrichtung 18 kann der Stab 32 bei der Implantation getrackt werden. An der Anzeigeeinheit 54 kann der Benutzer mit Hinweisen zum Führen des Implantationswerkzeuges 16 angeleitet werden. Es wird dabei davon ausgegangen, dass die Lage der Knochenschrauben 30 in dem durch die Markiereinrichtung 18 definierten Referenzkoordinatensystem bekannt ist. Beispielsweise ist eine der Knochenschrauben 30 mit einer in der Zeichnung nicht dargestellten medizinischen Markiereinrichtung versehen und die Position der anderen Knochenschrauben 30 relativ zu dieser Markiereinrichtung bekannt. Dadurch kann der Stab 32 beim Einsetzen relativ zu den Knochenschrauben 30 getrackt werden.

Es ist möglich, die Form des Stabes 32 präoperativ oder auch in situ zu ermitteln.

Figur 2 zeigt eine Verformungseinrichtung 22, um die Form des Stabes 32 zu verändern. Die Verformungseinrichtung 22 ist ausgestaltet als Biegeeinrichtung 62, insbesondere als handhaltbare und handbetätigbare Biegezange. Die Biegeeinrichtung 62 weist beispielsweise relativ zueinander verschwenkbare Branchen 64 auf. An einem distalen Ende 66 der Branchen können Anlageelemente 68 angeordnet sein zum Anlegen am Stab 32. Durch Handbetätigung kann der Stab 32 gebogen werden.

An der Biegeeinrichtung 62 ist vorzugsweise eine weitere medizinische Markiereinrichtung 24 angeordnet. Die Markiereinrichtung 24 kann entsprechend wie die Markiereinrichtung 18 vom Navigationssystem 20 im Raum verfolgt werden und stimmt in Funktion mit dieser überein.

Wenn die Form des Stabes 32, die anhand der Aufnahmen ermittelt worden ist, nicht mit der erforderlichen Form übereinstimmt, kann der Stab 32 durch Biegen mit der Biegeeinrichtung 62 in die gewünschte Form überführt werden. Zu diesem Zweck ist es zum Beispiel möglich, dass die Datenverarbeitungseinheit 48 an der Anzeigeeinheit 54 Hinweise für die Bedienperson bereitstellt, wie die Biegeeinrichtung 62 zum Biegen des Stabes 32 zu betätigen ist.

Das Biegen des Stabes 32 kann insbesondere in situ erfolgen. Von Vorteil ist es, wenn die Biegeeinrichtung 62 dabei über die Markiereinrichtung 24 vom Navigationssystem 20 verfolgt wird. Dies gibt die Möglichkeit, die Bedienperson insbesondere in situ zum Biegen des Stabes 32 anzuleiten. Ansatzpunkte der Anlageelemente 68 am Stab 32, die Biegeebene und der Biegeradius können durch Verfolgen der Biegeeinrichtung 62 relativ zum Stab 32 überprüft und verifiziert werden. Erforderlichenfalls können an der Anzeigeeinheit 54 Hinweise für die Bedienperson bereitgestellt werden, die Biegeeinrichtung 62 am Stab 32 zu repositionieren.

Anstelle der handbetätigten Verformungseinrichtung 22 kann auch eine maschinelle Verformungseinrichtung zum Einsatz kommen, wie dies bereits vorstehend erläutert wurde. Über eine Kommunikationsschnittstelle, zum Beispiel kabelgebunden oder kabellos, kann dieser Verformungseinrichtung eine Verformungsänderungsinformation bereitgestellt werden, damit die Verformungseinrichtung den Stab 32 in die gewünschte Form bringt.

### Bezugszeichenliste:

- 10: Instrumentarium
- 12: Stabilisierungselement
- 14: Fixationssystem
- 16: Implantationswerkzeug
- 18: Markiereinrichtung
- 20: Navigationssystem
- 22: Verformungseinrichtung
- 24: Markiereinrichtung
- 26: Wirbelkörper
- 28: Verankerungselement
- 30: Knochenschraube
- 32: Stab
- 34: Rigid body
- 36: Markierelement
- 38: Halterung
- 40: Distales Ende
- 42: Griffelement
- 44: Smartphone
- 46: Gehäuse
- 48: Datenverarbeitungseinheit
- 50: Kamera
- 52: Erfassungseinheit
- 54: Anzeigeeinheit
- 56: Beleuchtungseinheit
- 62: Biegeeinrichtung
- 64: Branchen
- 66: Distales Ende
- 68: Anlageelement

## Patentansprüche

1. Medizinisches Instrumentarium (10) mit einem Navigationssystem (20), das eine eine Kamera (50) aufweisende optische Erfassungseinheit (52) aufweist und eine mit dieser gekoppelte Datenverarbeitungseinheit (48), mit einem Stabilisierungselement (12) eines chirurgischen Fixationssystems (14) sowie einer medizinischen Markiereinrichtung (18), die in definierter räumlicher Anordnung am Stabilisierungselement (12) gehalten oder von diesem umfasst oder gebildet ist, wobei mit dem Navigationssystem (20) die Lage und Orientierung der Markiereinrichtung (18) im Raum ermittelbar ist, wobei mittels der Erfassungseinheit (52) zumindest zwei Aufnahmen des Stabilisierungselementes (12) und der Markiereinrichtung (18) unter unterschiedlicher Orientierung erstellbar sind und die Datenverarbeitungseinheit (48) so programmiert ist, dass sie die Form des Stabilisierungselementes (12) anhand der zwei oder mehr Aufnahmen ermittelt.

2. Instrumentarium nach Anspruch 1, **dadurch gekennzeichnet, dass** das Stabilisierungselement (12) ein Stab (32) ist.

3. Instrumentarium nach Anspruch 1 oder 2, **dadurch gekennzeichnet, dass** mehr als zwei Aufnahmen des Stabilisierungselementes (12) und der Markiereinrichtung erstellbar sind, anhand derer die Form des Stabilisierungselementes (12) ermittelbar ist, wobei die Orientierungen und Aufnahmen paarweise voneinander unterschiedlich sind.

4. Instrumentarium nach einem der voranstehenden Ansprüche, **dadurch gekennzeichnet, dass** das Navigationssystem (20) ein handhaltbares integriertes Navigationssystem (20) ist.

5. Instrumentarium nach Anspruch 4, **dadurch gekennzeichnet, dass** das Navigationssystem (20) ein Smartphone (44) oder ein Tabletcomputer ist.

6. Instrumentarium nach einem der voranstehenden Ansprüche, **dadurch gekennzeichnet, dass** das Navigationssystem (20) eine mit der Datenverarbeitungseinheit (48) gekoppelte Anzeigeeinheit (54) umfasst, an der die Aufnahmen des Stabilisierungselementes (12) darstellbar sind und/oder Hinweise für eine Bedienperson, die Aufnahmen durchzuführen und/oder eine Darstellung des Stabilisierungselementes (12), ermittelt anhand der Aufnahmen.

7. Instrumentarium nach einem der voranstehenden Ansprüche, **dadurch gekennzeichnet, dass** das Stabilisierungselement Markierelemente der Markiereinrichtung umfasst oder bildet, beispielsweise ausgestaltet als Punkte oder Linien am Stabilisierungselement.

8. Instrumentarium nach einem der voranstehenden Ansprüche, **dadurch gekennzeichnet, dass** die Markiereinrichtung am Stabilisierungselement lösbar festgelegt oder festlegbar ist.

9. Instrumentarium nach einem der voranstehenden Ansprüche, **dadurch gekennzeichnet, dass** die Markiereinrichtung einstückig mit dem Stabilisierungselement verbunden ist, insbesondere dass eine Sollbruchstelle vorgesehen ist zum Trennen der Markiereinrichtung vom Stabilisierungselement.

10. Instrumentarium nach einem der voranstehenden Ansprüche, **dadurch gekennzeichnet, dass** das Instrumentarium (10) ein Implantationswerkzeug (16) für das Stabilisierungselement (12) aufweist, an dem dieses und die Markiereinrichtung (18) gehalten sind.

11. Instrumentarium nach einem der voranstehenden Ansprüche, **dadurch gekennzeichnet, dass** das Instrumentarium (10) eine Verformungseinrichtung (22) aufweist, mit der die Form des Stabilisierungselementes (12) veränderbar ist, vorzugsweise dass die Verformungseinrichtung (22) eine Biegeeinrichtung (62) zum Biegen eines Stabes (32) ist, als der das Stabilisierungselement (12) ausgestaltet ist.

12. Instrumentarium nach Anspruch 11, **dadurch gekennzeichnet, dass** einer Bedienperson von der Datenverarbeitungseinheit (48) an einer Hinweiseinheit des Navigationssystems (20), beispielsweise an einer Anzeigeeinheit (54), Formänderungsinformationen zur Handhabung der Verformungseinrichtung (22) bereitstellbar sind, um das Stabilisierungselement (12) ausgehend von der ermittelten Form in eine vorgebbare Form zu bringen.

13. Instrumentarium nach Anspruch 11 oder 12, **dadurch gekennzeichnet, dass** vom Navigationssystem (20) über eine Kommunikationsschnittstelle Formänderungsinformationen an die Verformungseinrichtung (22) übertragbar sind, um das Stabilisierungselement (12) ausgehend von der ermittelten Form in eine vorgebbare Form zu bringen.

14. Instrumentarium nach einem der Ansprüche 11 bis 13, **dadurch gekennzeichnet, dass** das Instrumentarium (10) eine weitere medizinische Markiereinrichtung (24) umfasst, die in definierter räumlicher Anordnung an der Verformungseinrichtung (22) gehalten oder von dieser umfasst oder gebildet ist, dass mit dem Navigationssystem (20) die Lage und Orientierung der weiteren Markiereinrichtung (24) ermittelbar ist und dass einer Bedienperson mit der Datenverarbeitungseinheit (48) an einer Hinweiseinheit des Navigationssystems (20), beispielsweise an einer Anzeigeeinheit (54), Hinweise zum Führen der Verformungseinrichtung (22) bereitstellbar sind.

15. Verfahren zum Ermitteln der Form eines chirurgischen Stabilisierungselementes unter Einsatz eines Instrumentariums nach einem der voranstehenden Ansprüche mit einem Navigationssystem, das eine eine Kamera aufweisende optische Erfassungseinheit aufweist und eine mit dieser gekoppelte Datenverarbeitungseinheit, mit einem Stabilisierungselement eines chirurgischen Fixationssystems sowie einer medizinischen Markiereinrichtung, die in definierter räumlicher Anordnung am Stabilisierungselement gehalten oder von diesem umfasst oder gebildet ist, wobei mit dem Navigationssystem die Lage und Orientierung der Markiereinrichtung im Raum ermittelbar ist, wobei mittels der Erfassungseinheit zumindest zwei Aufnahmen des Stabilisierungselementes und der Markiereinrichtung unter unterschiedlicher Orientierung erstellt werden und die Form des Stabilisierungselementes von der Datenverarbeitungseinheit anhand der zwei oder mehr Aufnahmen ermittelt wird.

## Claims

1. Medical instrumentation (10) with a navigation system (20) which comprises an optical detection unit (52) comprising a camera (50), and a data processing unit (48) coupled to the detection unit, with a stabilization element (12) of a surgical fixation system (14) and with a medical marking device (18) which is held in a defined spatial arrangement on or is comprised by or formed by the stabilization element (12), the location and orientation of the marking device (18) in space being determinable with the navigation system (20), it being possible for at least two images of the stabilization element (12) and the marking device (18) to be taken from a different orientation by means of the detection unit (52), and the data processing unit (48) being programmed to determine the shape of the stabilization element (12) on the basis of the two or more images.

2. Instrumentation in accordance with claim 1, **characterized in that** the stabilization element (12) is a rod (32).

3. Instrumentation in accordance with claim 1 or 2, **characterized in that** it is possible for more than two images of the stabilization element (12) and the marking device to be taken, on the basis of which the shape of the stabilization element (12) is determinable, with the orientations and images differing from one another in pairs.

4. Instrumentation in accordance with any one of the preceding claims, **characterized in that** the navigation system (20) is a hand-held, integrated navigation system (20).

5. Instrumentation in accordance with claim 4, **characterized in that** the navigation system (20) is a smartphone (44) or a tablet computer.

6. Instrumentation in accordance with any one of the preceding claims, **characterized in that** the navigation system (20) comprises a display unit (54) which is coupled to the data processing unit (48) and on which it is possible to show the images of the stabilization element (12) and/or instructions for an operator for taking the images and/or a representation of the stabilization element (12), determined on the basis of the images.

7. Instrumentation in accordance with any one of the preceding claims, **characterized in that** the stabilization element comprises or forms marking elements of the marking device, for example, configured as points or lines on the stabilization element.

8. Instrumentation in accordance with any one of the preceding claims, **characterized in that** the marking device is releasably fixed or fixable to the stabilization element.

9. Instrumentation in accordance with any one of the preceding claims, **characterized in that** the marking device is integrally connected to the stabilization element, in particular **in that** a predetermined breaking point is provided for separating from the stabilization element.

10. Instrumentation in accordance with any one of the preceding claims, **characterized in that** the instrumentation (10) comprises an implantation tool (16) for the stabilization element (12), on which the stabilization element and the marking device (18) are held.

11. Instrumentation in accordance with any one of the preceding claims, **characterized in that** the instrumentation (10) comprises a reshaping device (22) with which the shape of the stabilization element (12) is changeable, preferably **in that** the reshaping device (22) is a bending device (62) for bending a rod (32), as which the stabilization element (12) is configured.

12. Instrumentation in accordance with claim 11, **characterized in that** it is possible for an operator to be provided by the data processing unit (48) on an indication unit of the navigation system (20), for example, on a display unit (54), with shape changing information for handling the reshaping device (22), in order to convert the stabilization element (12) from the determined shape into a prescribable shape.

13. Instrumentation in accordance with claim 11 or 12, **characterized in that** shape changing information is transferable from the navigation system (20) via a communication interface to the reshaping device (22), in order to convert the stabilization element (12) from the determined shape into a prescribable shape.

14. Instrumentation in accordance with any one of claims 11 to 13, **characterized in that** the instrumentation (10) comprises a further medical marking device (24) which is held in a defined spatial arrangement on or is comprised by or formed by the reshaping device (22), **in that** the location and orientation of the further marking device (24) is determinable with the navigation system (20), and **in that** it is possible for an operator to be provided by the data processing unit (48) on an indication unit of the navigation system (20), for example, on a display unit (54), with instructions for guiding the reshaping device (22).

15. Method for determining the shape of a surgical stabilization element using an instrumentation in accordance with any one of the preceding claims with a navigation system which comprises an optical detection unit comprising a camera, and a data processing unit coupled to the optical detection unit, with a stabilization element of a surgical fixation system and with a medical marking device which is held in a defined spatial arrangement on or is comprised by or formed by the stabilization element, the location and orientation of the marking device in space being determinable with the navigation system, wherein at least two images of the stabilization element and the marking device are taken from a different orientation by means of the detection unit and the shape of the stabilization element is determined by the data processing unit on the basis of the two or more images.

## Revendications

1. Ensemble d'instruments médicaux (10) avec un système de navigation (20), qui comporte une unité d'acquisition optique (52) comportant un appareil photographique (50) et une unité de traitement des données (48) couplée à celle-ci, avec un élément de stabilisation (12) d'un système de fixation chirurgical (14) ainsi qu'un dispositif de marquage médical (18) qui est maintenu dans une disposition spatiale définie sur l'élément de stabilisation (12) ou compris ou formé par celui-ci, où avec le système de navigation (20) la position et l'orientation du dispositif de marquage (18) dans l'espace peut être déterminée, où au moyen de l'unité d'acquisition (52) au moins deux prises de vue de l'élément de stabilisation (12) et du dispositif de marquage (18) peuvent être créées sous une orientation différente et l'unité de traitement des données (48) est programmée de telle manière qu'elle détermine la forme de l'élément de stabilisation (12) à l'aide des deux ou plusieurs prises de vue.

2. Ensemble d'instruments selon la revendication 1, **caractérisé en ce que** l'élément de stabilisation (12) est une tige (32).

3. Ensemble d'instruments selon la revendication 1 ou 2, **caractérisé en ce que** plus de deux prises de vue de l'élément de stabilisation (12) et du dispositif de marquage peuvent être créées, à l'aide desquelles la forme de l'élément de stabilisation (12) peut être déterminée, où les orientations et les prises de vue sont différentes les unes des autres par paires.

4. Ensemble d'instruments selon l'une des revendications précédentes, **caractérisé en ce que** le système de navigation (20) est un système de navigation (20) intégré pouvant être tenu à la main.

5. Ensemble d'instruments selon la revendication 4, **caractérisé en ce que** le système de navigation (20) est un smartphone (44) ou une tablette.

6. Ensemble d'instruments selon l'une des revendications précédentes, **caractérisé en ce que** le système de navigation (20) comprend une unité d'affichage (54) couplée à l'unité de traitement des données (48), sur laquelle les prises de vue de l'élément de stabilisation (12) peuvent être représentées et/ou des indications pour un opérateur pour réaliser les prises de vue et/ou une représentation de l'élément de stabilisation (12), déterminée à l'aide des prises de vue.

7. Ensemble d'instruments selon l'une des revendications précédentes, **caractérisé en ce que** l'élément de stabilisation comprend ou forme des éléments de marquage du dispositif de marquage, par exemple conçus sous forme de points ou de lignes sur l'élément de stabilisation.

8. Ensemble d'instruments selon l'une des revendications précédentes, **caractérisé en ce que** le dispositif de marquage est fixé ou peut être fixé de manière amovible sur l'élément de stabilisation.

9. Ensemble d'instruments selon l'une des revendications précédentes, **caractérisé en ce que** le dispositif de marquage est relié d'une pièce à l'élément de stabilisation, en particulier **en ce qu'**un point destiné à la rupture est prévu pour la séparation du dispositif de marquage d'avec l'élément de stabilisation.

10. Ensemble d'instruments selon l'une des revendications précédentes, **caractérisé en ce que** l'ensemble d'instruments (10) comporte un outil d'implantation (16) pour l'élément de stabilisation (12) sur lequel celui-ci et le dispositif de marquage (18) sont maintenus.

11. Ensemble d'instruments selon l'une des revendications précédentes, **caractérisé en ce que** l'ensemble d'instruments (10) comporte un dispositif de déformation (22), avec lequel la forme de l'élément de stabilisation (12) est modifiable, de préférence **en ce que** le dispositif de déformation (22) est un dispositif de cintrage (62) pour cintrer une tige (32) sous forme de laquelle l'élément de stabilisation (12) est conçu.

12. Ensemble d'instruments selon la revendication 11, **caractérisé en ce que** des informations de changement de forme pour manipuler le dispositif de déformation (22) peuvent être fournies à un opérateur par l'unité de traitement des données (48) sur une unité d'indication du système de navigation (20), par exemple sur une unité d'affichage (54), pour amener l'élément de stabilisation (12) de la forme déterminée dans une forme pouvant être prédéfinie.

13. Ensemble d'instruments selon la revendication 11 ou 12, **caractérisé en ce que** des informations de changement de forme peuvent être transférées du système de navigation (20) par l'intermédiaire d'une interface de communication au dispositif de déformation (22) pour amener l'élément de stabilisation (12) dans une forme pouvant être prédéfinie à partir de la forme déterminée.

14. Ensemble d'instruments selon l'une des revendications 11 à 13, **caractérisé en ce que** l'ensemble d'instruments (10) comprend un autre dispositif de marquage médical (24) qui est maintenu dans une disposition spatiale définie sur le dispositif de déformation (22) ou compris ou formé par celui-ci, **en ce qu'**avec le système de navigation (20) l'emplacement et l'orientation de l'autre dispositif de marquage (24) peuvent être déterminés et **en ce que** des indications pour diriger le dispositif de déformation (22) peuvent être fournies à un opérateur avec l'unité de traitement des données (48) sur une unité d'indication du système de navigation (20), par exemple sur une unité d'affichage (54).

15. Procédé pour déterminer la forme d'un élément de stabilisation chirurgical à l'aide d'un ensemble d'instruments selon l'une des revendications précédentes avec un système de navigation qui comporte une unité d'acquisition optique comportant un appareil photographique et une unité de traitement des données couplée à celle-ci, avec un élément de stabilisation d'un système de fixation chirurgical ainsi qu'avec un dispositif de marquage médical qui est maintenu dans une disposition spatiale définie sur l'élément de stabilisation ou est compris ou formé par celui-ci, où avec le système de navigation l'emplacement et l'orientation du dispositif de marquage dans l'espace peuvent être déterminés, où au moyen de l'unité d'acquisition au moins deux prises de vue de l'élément de stabilisation et du dispositif de marquage sont créées sous une orientation différente et la forme de l'élément de stabilisation est déterminée par l'unité de traitement des données à l'aide des deux ou plusieurs prises de vue.
